# EUROPEAN PATENT SPECIFICATION

(11) **EP 0 772 428 B1**
(45) Date of publication and mention of the grant of the patent: **04.04.2001**
(21) Application number: 95925937.5
(22) Date of filing: 21.07.1995
(51) Int. Cl.: A61F 9/00

(54) **DEVICE FOR MANIPULATING OR HOLDING DELICATE OBJECTS**
GERÄT ZUR HANDHABUNG UND ZUM HALTEN VON ZARTEN GEGENSTÄNDEN
DISPOSITIF PERMETTANT DE MANIPULER OU TENIR DES OBJETS DELICATS

(30) Priority: 22.07.1994 GB 9414771; 10.04.1995 GB 9507403
(43) Date of publication of application: 14.05.1997
(73) Proprietor: Sheldon, Mark Daniel, London NW6 7NG (GB)
(72) Inventor: Sheldon, Mark Daniel, London NW6 7NG (GB)
(74) Representative: Barnard, Eric Edward
(86) International application number: GB9501722
(87) International publication number: WO9603099

(56) References cited:
- EP-A- 0 490 301
- CA-A- 1 103 119
- US-A- 3 503 397
- US-A- 4 120 302
- US-A- 4 126 345
- US-A- 4 245 859
- US-A- 4 452 244
- US-A- 4 834 090
- US-A- 4 877 280
- US-A- 4 964 663
- US-A- 4 986 586

## Description

### TECHNICAL FIELD

The present invention relates to a device for use in manipulating or holding delicate objects, such as contact lenses, and particularly for use in the removal and subsequent handling of soft contact lenses.

### BACKGROUND OF THE INVENTION

Users of contact lenses normally have to remove their lenses by pinching each lens in turn between a thumb and a finger gently to squeeze the lens sufficiently to deform the lens outwardly from the eye thereby to detach the lens. Similarly, when inserting a contact lens the user positions the lens with a finger and introduces the lens into the eye. It is possible for a contact lens or even the eye to be damaged by the user's finger nails or rough skin during the insertion or removal procedure and a general object of the invention is to provide a device usable to avoid these difficulties. Devices usable for manipulating or holding delicate objects, such as contact lenses, are known. US-A-4986586 describes one such device which is composed of a member with spaced-apart arms which can be moved together or apart. The arms have free ends each of which mounts a sleeve which supports an elongate reed-like member carrying a one-piece soft tip. US-A-4126345 describes another similar device in which the arms are in the form of leaf springs and end regions of the arms are surrounded by tips formed of a soft material which can only deform to a limited extent due to the presence of the end regions of the arms.

### DISCLOSURE OF THE INVENTION

As is known from US-A-4986586 and US-A-4126345, a device constructed in accordance with the present invention is composed of a member, conveniently a U or V shaped member, with spaced-apart arms which can be moved together or apart and resilient finger elements mounted on the arms for contracting an object to be held by the device. In contrast to US-A- 4986586 and US-A-4126345, each finger element has one end mounted at a free end of the associated arm to the extent that the free end of the associated arm does not extend beyond the one end of the finger element and a shaped wall extending from the one end. A hollow space can be defined interiorly of the wall or the space interiorly of the wall can be at least partly filled with plastics foam material. Each finger element is made from a material which is softer and more resilient than the member so that the entire walls of the finger elements or the finger elements with the foam filling are easily deformed when exposed to force and ensure no excessive force can be exerted on an object held between the finger elements when the arms are moved together.

The finger elements can be filled with gas, such as air under pressure, to promote resilience.

The arms of the device can be gripped manually and used to move the finger elements together or apart and at least part of the arms may have a non smooth exterior surface for gripping by the user.

The finger elements are primarily intended to contact a delicate object such as a contact lens and to trap the object or contact lens therebetween. Since the finger elements are softer and more resilient and more deformable than the stiffer arms of the member these elements can then exert only minimal force on the object or contact lens with no likelihood of damage.

Conveniently in one embodiment of the invention, the arms are joined directly together opposite their free ends and the material from which the member is made is inherently resilient to hold the finger element apart. In other embodiments however, the arms can be flexibly or pivotably connected and a separate resilient spring means can hold the arms apart. The arms can have a rectangular cross-section. The arms can be squeezed together manually to grip the object with the finger elements and resilient force biases the arm apart. It is possible to provide means for limiting the relative movement of the arms towards one another.

The finger element may be made from rubber, natural or synthetic, with a non-smooth roughened or textured exterior surface created by moulding scoring or etching for example.

The walls of the finger element may be continuous in which case the free ends of the arms have vents lading to the interiors of the finger elements. The walls of the finger elements can also be perforated.

The finger elements may be shaped like nipples with rounded or bulbous end regions which can deform to some extent when exerting holding force on the object or the contact lens. The radius of curvature of the end regions is preferably commensurate with the size of a human eye and/or a contact lens.

The finger elements of the device are preferably designed to simulate the user's fingers with regard to the tactile feel and rolling resistance. The use of the device for the insertion and removal of contact lenses is safer than the use of the user's fingers and the user can view the finger elements of the device and the contact lens held thereby more easily than when the contact lens is inserted or removed manually direct The use of the device is also more hygenic than the conventional technique.

Removable protective caps or a sheath can be provided to surround the exposed portions of the finger elements and to keep them clean.

It is feasible to have the finger elements in one-piece with the arms even if the finger elements are made separately and fixed in place but according to a further feature of this invention the finger elements are detachably secured to the arms of the member. A pair of screw-threaded complementary components can lock the finger elements to the arms or can be released to permit the finger elements to be removed, for example, for separate cleaning or disposal and replacement. Alternatively the finger elements can be detachably locked to the arms by providing a snap-fit connection therebetween.

The invention may be understood more readily, and various other aspects and features of the invention may become apparent, from consideration of the following description.

### BRIEF DESCRIPTION OF DRAWINGS

Embodiments of the invention will now be described, by way of examples only with reference to the accompanying drawing wherein:
Figure 1 is a side view of a device constructed in accordance with the invention;
Figure 2 is a front view of the device shown in Figure 1;
Figure 3 is a part sectional end view of the device shown in Figures 1 and 2, the view being taken along the line III-III in Figure 1;
Figure 4 is a part sectional side view of part of the device shown in Figures 1 to 3 and taken on a somewhat larger scale;
Figure 5 is a part-sectional side view of another device constructed in accordance with the invention;
Figure 6 is a side view of a separate finger element of the device shown in Figure 5;
Figure 7 is a sectional side view of the finger element shown in Figure 6;
Figure 8 is a side view of another separate finger element for use in the device shown in Figure 5;
Figure 9 is a sectional side view of the finger element shown in Figure 8;
Figure 10 is a side view of another separate finger element for use in the device shown in Figure 5;
Figure 11 is a sectional side view of the finger element shown in Figure 10;
Figure 12 is a side view of another separate finger element for use in the device shown in Figure 5;
Figure 13 is a sectional side view of the finger element shown in Figure 12;
Figure 14 is a side view of a further separate finger element for use in the device shown in Figure 5,
Figure 15 is a sectional side view of the finger element shown in Figure 14, and
Figure 16 is a perspective view of the device shown in Figure 5 in combination with a protective sheath.

### BEST MODES FOR CARRYING OUT THE INVENTION

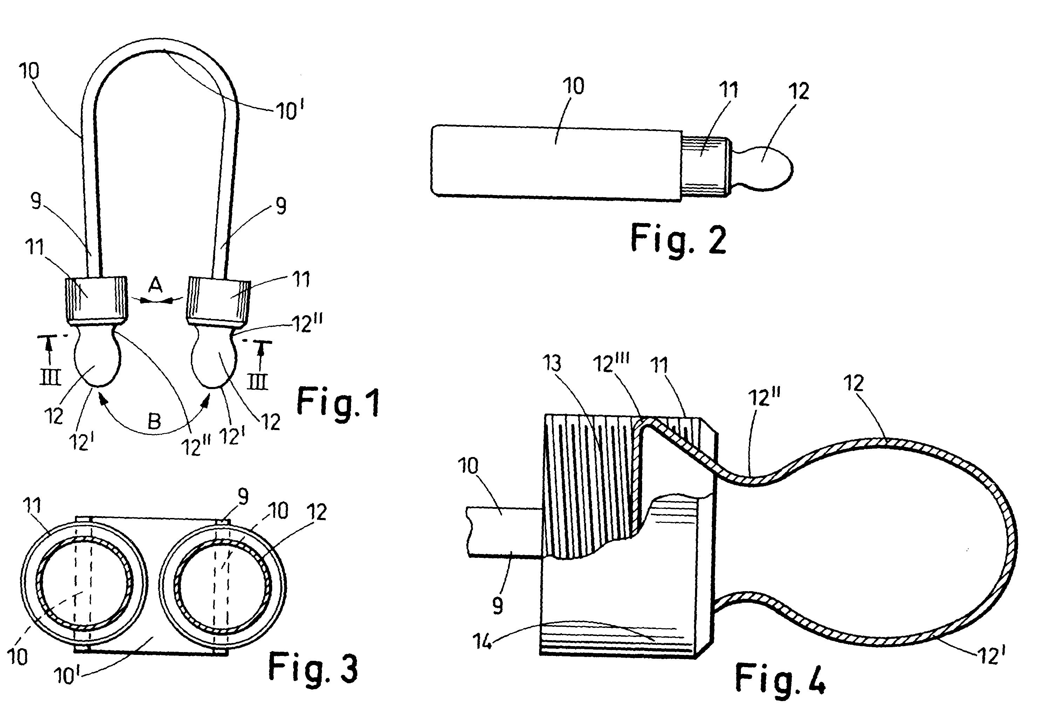

As shown in Figures 1 to 4 of the drawings, a device constructed in accordance with the invention is composed of a U-shaped member 10 with spaced apart arms 9 composed of a suitable resilient plastics material such as ABS, acrylic, PC, SAN, PA, PP, PET or PBT. The member 10 is solid and conveniently of rectangular cross-section to bias its resilient action like a leaf spring.

The free ends of the arms 9 of the member 10 opposite the U-shaped joining region 10' are provided with mounting pieces 11, 13 which locate detachable finger elements 12. As shown in Figure 4, the mounting pieces 11, 13 are composed of screw-threaded cylindrical blocks 13 fixed to the ends of the arms 9 of the member 10 and complementary screw-threaded ferrules 11 which can be screwed onto or unscrewed from the blocks 13. Each finger element 12 is hollow and has the shape of a nipple with a bulbous outer end region 12' a narrow waist region 12" and an outwardly flared inner region 12"'. The narrow region 12" engages through an aperture in an end wall of the associated ferrule 11 so that the element 12 is trapped by the inner region 12"' within the ferrule 11. The elements 12 are detachably fitted to the member 10. Preferably, the finger elements 12 have textured non-smooth or roughened external surface. The material of the elements 12 can be absorbent of liquids, porous or non-porous. The material of the elements 12 can be hypoallergenic and Ph neutral. The elements 12 can be filled with another material or with gas. A preferable material for the elements 12 is natural latex rubber although pvc, silicone and urethane foams can be used. The radius of curvature of the end regions 12' of the elements 12 is generally such as to match an average human eye size. Although size can vary typical dimensions for the elements 12 are maximum diameter in the range 6 to 14mm and maximum length 15 to 20mm.

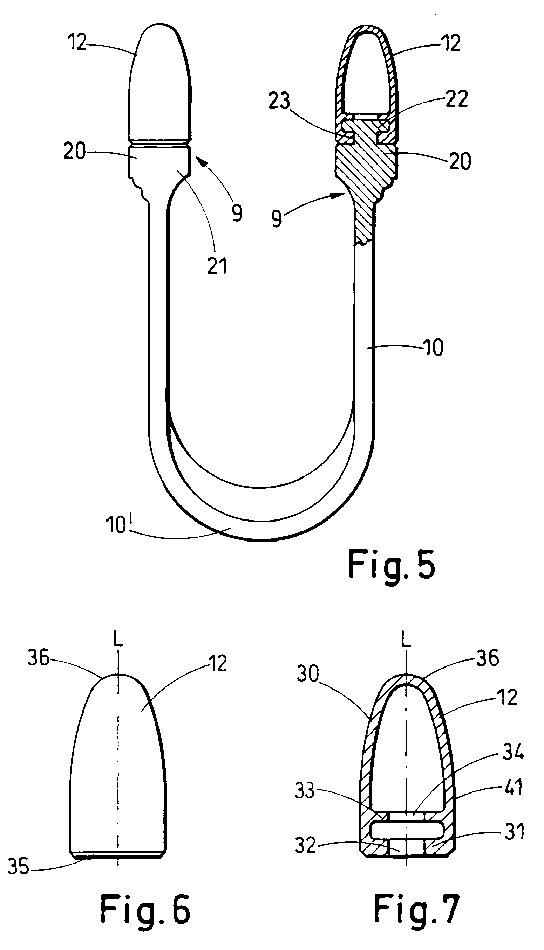

Figures 5 to 7 depict another embodiment of the device where like reference numerals denote like parts to Figures 1 to 4. In this other embodiment the free ends of the arms 9 have enlarged integral end regions 20 which serve as mounting pieces for the finger elements 12. As shown each end region 20 flares smoothly out from the adjoining part of the arm 9 to form a cylindrical body 21. A flat head 22 of smaller diameter than the body 21 is separated from the body 21 by a groove 23. The associated finger elements 12 are designed to engage onto the end regions 20 as a detachable snap fit. As shown in Figures 6 and 7, each finger element 12 is hollow and symmetrical about a longitudinal axis L. The finger element 12 has a main wall 30. The wider base of the finger element 12 has a radially inwardly extending wall 31 with an aperture 32. A further internal wall 33 spaced axially from the wall 31 has an aperture 34.

The main wall 30 tapers smoothly and progressively inwardly from a cylindrical base region 41 near the base wall 31 to provide a curved end or tip 36. The transition between the main wall 30 and the base wall 31 can be chamfered (as at 35 Figure 6)

The inherent resilience of the finger element 12 permits the base of the finger element 12 to be pressed onto the end region 20 of one of the arms 9 so that the wall 31 snaps into the groove 23 while the head 22 engages between the walls 31,33 as shown in the sectional part of Figure 5.

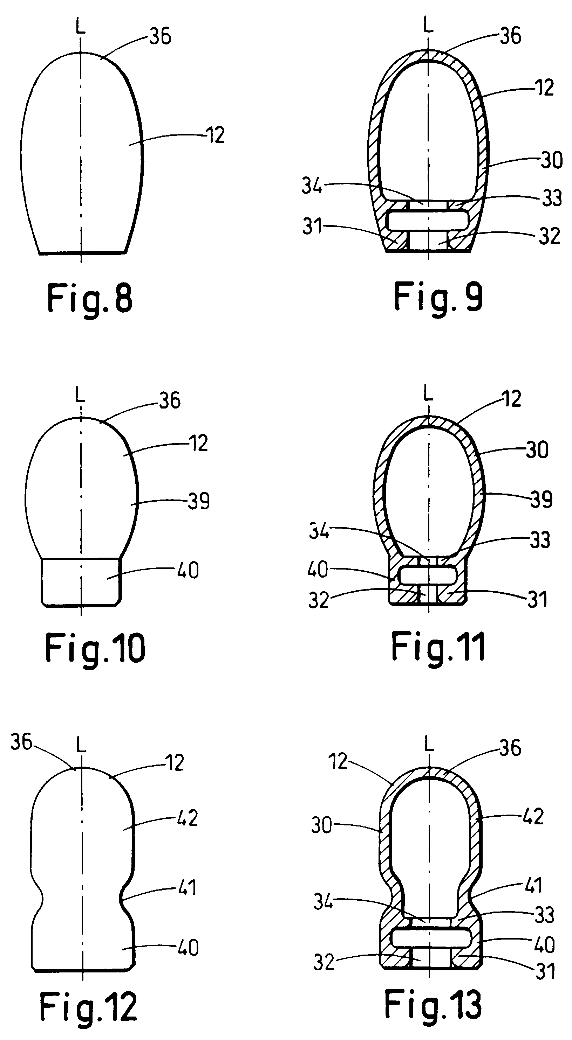

Figures 8 to 15 depict further forms of the finger element 12 where like reference numerals denote like parts to Figures 6 and 7.

The wall 30 of the finger element 12 depicted in Figures 8 and 9 is generally bulbous overall whilst the wall 30 of the finger element 12 depicted in Figures 10 and 11 has a bulbous region 39 and the cylindrical base region 40.

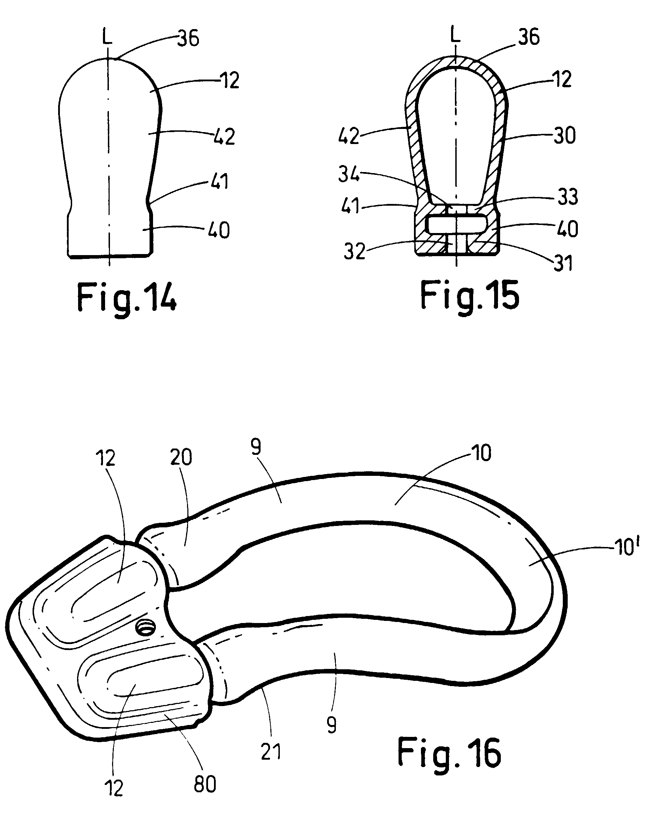

The wall 30 of the finger element 12 depicted in Figures 12 and 13 has an inwardly projecting recessed region 41 between a base region 40 and a more regular upper region 42. The wall of the finger element 12 depicted in Figures 14 and 15 also has a somewhat smaller recessed region 41 between a base region 40 and an outwardly flaring upper region 42.

In further modified designs of the finger elements 12 the wall 30 is not symmetrical about the axis of symmetry but there is an angular offset between the axis L and the tip 36.

During use of the devices as described, the arms 9 of the member 10 can be displaced manually towards one another in the direction of arrow A in Figures 1 and 5 and tend to move apart in the direction of arrow B due to the inherent resilience of the member 10. This enables the elements 12 to be used to engage or release a contact lens. A contact lens can be trapped between the elements 12 for insertion into or removal from a person's eye. The U-shaped joining region 10' of the member 10 in the embodiment depicted in Figure 5 can be additionally shaped to accommodate the cheek bone of a user. It is also desirable to shape the member 10 so that when the device is placed onto a supporting surface the finger elements 12 do not contact the supporting surface. A further shaping of the member 10 can allow access for the user's index or middle finger to draw down his lower eyelid to assist in removal of the contact lens. It is desirable to provide a non-smooth frictional texture to the arms 9 or to part of the arms 9 for better gripping by the user.

Although the finger elements 12 are best inherently soft and deformable so they cannot apply excessive force to the contact lens or object trapped therebetween it is possible to modify the device so that the arms 9 of the member 10 can only be moved inwardly to a set distance apart. This will then preclude the possibility of applying too much force to the contact lens. This control means for the arms 9 can be by way of a mechanical stop or by selecting the inherent resilience of the material from which the member 10 is made so that the user will experience a high force when the arms 9 are moved together beyond a set distance. It may be desirable also to provide some adjustment of the pre-determined minimum space between the ends of the arms 9.

The construction and shape for the finger elements 12 as depicted permits the walls of the elements 12 to deform and also permits the elements 12 to deform about their longitudinal axes L. This has been found particularly effective in gently squeezing the contact lens to a sufficient extent to enable the lens to be held reliably between the elements 12. The wall 30 of each finger element 12 may be perforated to allow air to pass into and from the element 12 as it is deformed. Alternatively, the arms 9 can be provided with apertures or slots leading into the elements 12 to permit air to pass in and out. The size of the apertures or slots can be designed to control the deformation of the finger elements.

In a further modification the material of the finger element 12 has a strong contrast, say a fluorescent colour, contrasting with the member 10 to enable a user to observe the elements 12 better as the elements 12 approach the user's eye to make physical contact with the contact lens therein or to place the contact lens in position.

The mounting pieces at the ends of the arms 9 can also be modified or adapted so that capsules containing a suitable cleansing liquid can be fitted onto the arms 9 around the finger elements 11 and sealed. In this way the finger elements 12 can be immersed into the cleansing liquid when the device is not in use and the capsules can be removed when the device is to be used for contact lens removal or insertion. It is also possible to rely on other ways of cleaning the finger elements 12 and indeed these can be replaced from time to time. The provision of empty caps or capsules surrounding the finger elements 12 and fitted again as with a snap fit for example, onto the arms 9 can be useful without the use of cleansing liquid to protect the finger elements 12 and to isolate the finger elements 12 from the surrounding atmosphere. Figure 16 depicts a sheath or casing 80 which surrounds the finger element 12 to protect these.

In a further modification a small sheet or bag can be provided as an accessory which is detachably fitted onto the devices as illustrated and described so that should a contact lens become accidentally detached from the grip of the finger elements 12 it will be caught in the sheet or bag.

Although the finger elements 12 are shown as separate and detachable from the carrier means or member 10 it is possible to fix the elements 12 in place or even to fabricate the finger elements 12 in one piece with the carrier means 10.

## Claims

1. A device for use in manipulating or holding delicate objects, such as contact lenses;
said device being composed of a member (10) with spaced-apart arms (9) which can be moved together or apart and resilient finger elements (12) mounted on the arms (9) for contacting an object to be held by the device;
characterised in that each finger element has one end mounted at a free end of the associated arm (9) to the extent that the free end of the associated arm does not extend beyond the one end of the finger element and the finger element has a shaped wall extending outwardly from the one end which defines a space interiorly of the wall the space being entirely hollow or at least partly filled with plastics foam material and each finger element is made from a material which is softer and more resilient than the member so that the entire walls of the finger elements are easily deformed when exposed to force and ensure no excessive force can be exerted on an object held between the finger elements when the arms are moved together.

2. A device according to claim 1, wherein means is provided to allow air to pass into and out from the finger elements (12).

3. A device according to claim 1 or 2, wherein the finger elements (12) have a non-smooth textured exterior surface.

4. A device according to any one of claims 1 to 3, wherein the shaped wall of each of the finger elements (12) has a rounded or bulbous end region (12') remote from the one end.

5. A device according to claim 4, wherein the radius of curvature of the end regions is commensurate with the size of a human eye and/or a contact lens.

6. A device according to any one or more of claims 1 to 5, wherein the finger elements (12) are detachably secured to the arms (9).

7. A device according to any one or more of claims 1 to 6, wherein the finger elements (12) are detachably fitted to the arms with a snap-fitting releasable connection.

8. A device according to any one or more of claims 1 to 7, wherein at least part of the arms (9) of the member (10) have a non smooth exterior surface for gripping by a user.

9. A device according to any one or more of claims 1 to 8, wherein the member (10) with the arms (9) is made from an inherently resilient plastics material and the finger elements are resiliently biased apart.

10. A device according to any one or more of claims 1 to 8, wherein the arms (9) of the member are biased apart by resilient spring means to hold the finger elements (12) apart.

11. A device according to any one or more of claims 1 to 10, wherein the cross-section of at least part of each of the arms (9) is rectangular.

12. A device according to any one or more of claims 1 to 11, and further comprising means for limiting the relative movement of the arms (9) towards one another.

13. A device according to any one or more of claims 1 to 12, wherein the finger elements (12) are made from natural or synthetic rubber.

14. A device according to any one or more of claims 1 to 13, in combination with a sheath (20) or caps which fit over the finger elements (12) to protect the finger elements (12) from the surroundings.

## Patentansprüche

1. Vorrichtung zur Verwendung bei der Handhabung oder beim Festhalten empfindlicher Objekte, wie zum Beispiel Kontaktlinsen;
wobei die Vorrichtung aus einem Glied (10) mit voneinander beabstandeten Armen (9), die aufeinander zu- oder voneinander weg bewegt werden können, sowie aus an den Armen (9) befestigten elastischen Fingerelementen (12) zur Berührung eines durch die Vorrichtung festzuhaltenden Objektes besteht; dadurch gekennzeichnet, daß ein Ende jedes Fingerelements (12) so an einem freien Ende des dazugehörigen Arms (9) angebracht ist, daß sich das freie Ende des dazugehörigen Arms nicht über das eine Ende des Fingerelements hinaus erstreckt, und das Fingerelement eine geformte Wand aufweist, die sich von dem einen Ende nach außen erstreckt und innerhalb der Wand einen Raum definiert, wobei der Raum ganz hohl oder mindestens teilweise mit Schaumkunststoff gefüllt ist und jedes Fingerelement aus einem Material besteht, das weicher und elastischer ist als das Glied, so daß die gesamten Wände der Fingerelemente leicht verformt werden, wenn auf sie eine Kraft ausgeübt wird, und gewährleisten, daß auf ein zwischen den Fingerelementen festgehaltenes Objekt keine übermäßige Kraft ausgeübt werden kann, wenn die Arme aufeinander zu bewegt werden.

2. Vorrichtung nach Anspruch 1, bei der ein Mittel vorgesehen ist, das das Ein- und Ausströmen von Luft in die bzw. aus den Fingerelementen (12) gestattet.

3. Vorrichtung nach Anspruch 1 oder 2, bei der die Fingerelemente (12) eine Außenfläche mit nicht-glatter Oberflächenbeschaffenheit aufweisen.

4. Vorrichtung nach einem der Ansprüche 1 bis 3, bei der die geformte Wand jedes der Fingerelemente (12) einen von dem einen Ende entfernten abgerundeten oder bulboiden Endbereich (12') aufweist.

5. Vorrichtung nach Anspruch 4, bei der der Krümmungsradius der Endbereiche der Größe eines menschlichen Auges und/oder einer Kontaktlinse entspricht.

6. Vorrichtung nach einem oder mehreren der Ansprüche 1 bis 5, bei der die Fingerelemente (12) an den Armen (9) lösbar befestigt sind.

7. Vorrichtung nach einem oder mehreren der Ansprüche 1 bis 6, bei der die Fingerelemente (12) mittels einer lösbaren Schnappverbindung an den Armen lösbar befestigt sind.

8. Vorrichtung nach einem oder mehreren der Ansprüche 1 bis 7, bei der zumindest ein Teil der Arme (9) des Glieds (10) eine nichtglatte Außenfläche als Griffläche für einen Benutzer aufweist.

9. Vorrichtung nach einem oder mehreren der Ansprüche 1 bis 8, bei der das Glied (10) mit den Armen (9) aus einem in sich elastischen Kunststoffmaterial besteht und die Fingerelemente elastisch auf Abstand vorgespannt sind.

10. Vorrichtung nach einem oder mehreren der Ansprüche 1 bis 8, bei der die Arme (9) des Glieds durch ein elastisches Federmittel voneinander weg vorgespannt sind, um die Fingerelemente (12) auf Abstand zu halten.

11. Vorrichtung nach einem oder mehreren der Ansprüche 1 bis 10, bei der der Querschnitt von zumindest einem Teil jedes der Arme (9) rechteckig ist.

12. Vorrichtung nach einem oder mehreren der Ansprüche 1 bis 11, die weiterhin ein Mittel zur Begrenzung der Relativbewegung der Arme (9) aufeinander zu umfaßt.

13. Vorrichtung nach einem oder mehreren der Ansprüche 1 bis 12, bei der die Fingerelemente (12) aus Natur- oder synthetischem Gummi bestehen.

14. Vorrichtung nach einem oder mehreren der Ansprüche 1 bis 13, in Kombination mit einer Hülle (20) oder Kappen, die über die Fingerelemente (12) passen, um die Fingerelemente (12) vor der Umgebung zu schützen.

## Revendications

1. Dispositif permettant de manipuler ou tenir des objets délicats, tels que des verres de contact;
ledit dispositif étant composé d'un membre (10) ayant deux bras (9) écartés, qui peuvent être déplacés ensemble ou séparément, et d'éléments résilients en forme de doigt (12) fixés sur les bras (9) pour être en contact avec un objet destiné à être tenu par le dispositif;
caractérisé en ce que chaque élément en forme de doigt a une extrémité fixée à l'extrémité libre du bras associé (9), l'extrémité libre du bras associé ne s'étendant pas au-delà de l'extrémité de l'élément en forme de doigt, et en ce que l'élément en forme de doigt a une paroi façonnée, s'étendant vers l'extérieur à partir de l'extrémité, qui définit un espace à l'intérieur de la paroi, l'espace étant entièrement creux ou au moins partiellement rempli d'une mousse en matière plastique, et en ce que chaque élément en forme de doigt est fait à partir d'une matière qui est plus molle et plus résiliente que le membre de sorte que les parois entières des éléments en forme de doigt soient aisément déformées quand elles sont soumises à une force et assurent qu'aucune force excessive ne peut être exercée sur un objet maintenu entre les éléments en forme de doigt lorsque les bras sont déplacés ensemble.

2. Un dispositif selon la revendication 1, dans lequel il est prévu un moyen destiné à permettre l'entrée ou la sortie d'air des éléments en forme de doigt (12).

3. Un dispositif selon la revendication 1 ou 2, dans lequel les éléments en forme de doigt (12) ont une surface extérieure de texture non lisse.

4. Un dispositif selon l'une quelconque des revendications 1 à 3, dans lequel la paroi façonnée de chacun des éléments en forme de doigt (12) a une terminaison (12'), ronde ou en forme de bulbe, éloignée de l'extrémité.

5. Un dispositif selon la revendication 4, dans lequel le rayon de courbure des terminaisons est proportionné à la taille d'un oeil humain et/ou d'un verre de contact.

6. Un dispositif selon l'une quelconque ou plus des revendications 1 à 5, dans lequel les éléments en forme de doigt (12) sont fixés, tout en étant amovibles, aux bras (9).

7. Un dispositif selon l'une quelconque ou plus des revendications 1 à 6, dans lequel les éléments en forme de doigt (12) sont fixés, tout en étant amovibles, aux bras à l'aide d'une connexion détachable permettant une fixation par encliquetage.

8. Un dispositif selon l'une quelconque ou plus des revendications 1 à 7, dans lequel au moins une partie des bras (9) du membre (10) a une surface extérieure non lisse pour être saisie par l'utilisateur.

9. Un dispositif selon l'une quelconque ou plus des revendications 1 à 8, dans lequel le membre (10) avec les bras (9) sont faits d'une matière plastique présentant la propriété d'être résiliente en soi, et les éléments en forme de doigt sont élastiquement maintenus éloignés.

10. Un dispositif selon l'une quelconque ou plus des revendications 1 à 8, dans lequel les bras (9) du membre (10) sont élastiquement maintenus éloignés à l'aide de moyens formant ressort résilient permettant de tenir séparés les éléments en forme de doigt (12).

11. Un dispositif selon l'une quelconque ou plus des revendications 1 à 10, dans lequel la coupe transversale d'au moins une partie de chacun des bras (9) est rectangulaire.

12. Un dispositif selon l'une quelconque ou plus des revendications 1 à 11, et comprenant en outre des moyens limitant le mouvement relatif des bras (9) l'un vers l'autre.

13. Un dispositif selon l'une quelconque ou plus des revendications 1 à 12, dans lequel les éléments en forme de doigt (12) sont faits de caoutchouc synthétique ou naturel.

14. Un dispositif selon l'une quelconque ou plus des revendications 1 à 13, en combinaison avec une enveloppe (20) ou chapeaux qui s'enfilent sur les éléments en forme de doigt (12) pour protéger les éléments en forme de doigt (12) de l'environnement.
